# EUROPÄISCHE PATENTANMELDUNG

(11) **EP 1 595 874 A1**
(43) Veröffentlichungstag der Anmeldung: **16.11.2005**
(21) Anmeldenummer: 05009559.5
(22) Anmeldetag: 30.04.2005
(51) Int. Cl.: C07D 303/22, C07D 303/24, C07C 43/178

(54) **Pumpbare Tricyclodecandimethylol enthaltende Zusammensetzung**

(30) Priorität: 11.05.2004 DE 102004023071
(71) Anmelder: BASF Aktiengesellschaft, 67056 Ludwigshafen (DE)
(72) Erfinder: Wittkowski, Lars, Dr., 68167 Mannheim (DE); Papp, Rainer, Dr., 67346 Speyer (DE)

(57) **Zusammenfassung**

Zusammensetzung enthaltend Tricyclodecandimethylol und mindestens eine polymerisierbare Verbindung, bevorzugt ausgewählt aus (Meth)acrylsäure, deren Estern, O- und N-Vinylverbindungen, Epichlorhydrin, C₁- bis C₁₀-Alkylenoxiden, in einem Mengenverhältnis von 100:1 bis 1:1.

## Beschreibung

Die Erfindung betrifft eine pumpbare Tricyclodecandimethylol enthaltende Zusammensetzung, welche zusätzlich polymerisierbare Verbindungen aufweist.

Tricyclodecandimethylol (im folgendenTCD-Dimethylol genannt) ist ein verbreiteter Rohstoff für die Herstellung von Acrylaten oder Epoxidharzen. Die daraus hergestellten Polyacrylate und Epoxidharze werden unter anderem in Farben und Coatings mit guter Adhäsion, hoher Hitze-, Wetter- und Schlagbeständigkeit eingesetzt.

TCD-Dimethylol in handelsüblicher Qualität ist schon bei Raumtemperatur so hochviskos, dass es nicht ohne weiteres pumpfähig ist. Hersteller von Folgeprodukten sind daher gezwungen, TCD-Dimethylol zunächst aufzuschmelzen, um es dann aus dem Liefergebinde pumpen zu können. Dieser Zeit- und Energiebedarf ist unwirtschaftlich.

Aufgabe der vorliegenden Erfindung ist es, diesen Nachteil zu vermeiden.

Überraschenderweise wurde nun gefunden, dass eine Zusammensetzung enthaltend Tricyclodecandimethylol (I) 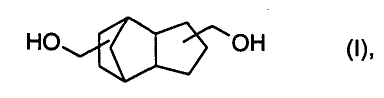 und mindestens eine polymerisierbare, bevorzugt eine mindestens einfach ungesättigte, Verbindung in einem Gewichtsverhälthis von 100:1 bis 1:1, bevorzugt 20:1 bis 3:1, bei Raumtemperatur ohne Aufschmelzen pumpbar ist. Vorteilhaft ist weiterhin, dass es bei dieser Temperatur nicht zu Reaktionen, wie z. B. Polymerisationen kommt. Unter Raumtemperatur wird eine Temperatur von 15 bis 30 °C verstanden.

Die polymerisierbare Verbindung ist bevorzugt ausgewählt aus Methacryl- oder Acrylsäure (im folgenden (Meth)acrylsäure genannt), deren Estern, O- und N-Vinylverbindungen, C₁- bis C₁₀-Alkylenoxiden wie zum Beispiel Ethylenoxid, Propylenoxid und Epichlorhydrin.

Die einsetzbare (Meth)acrylsäure ist nicht beschränkt und kann im Fall von Roh-(Meth)acrylsäure beispielsweise folgende Komponenten aufweisen:

| | |
|---|---|
| (Meth)acrylsäure | 90 - 99,9 Gew% |
| Essigsäure | 0,05 - 3 Gew% |
| Propionsäure | 0,01 - 1 Gew% |
| Diacrylsäure | 0,01 - 5 Gew% |
| Wasser | 0,05 - 5 Gew% |
| Aldehyde | 0,01 - 0,3 Gew% |
| Inhibitoren | 0,01 - 0,1 Gew% |
| Maleinsäure(-anhydrid) | 0,001 - 0,5 Gew% |

Die eingesetzte Roh-(Meth)acrylsäure ist in der Regel stabilisiert mit 200 - 600 ppm Phenothiazin oder anderen Stabilisatoren in Mengen, die eine vergleichbare Stabilisierung ermöglichen.

Unter Roh-(Meth)acrylsäure wird hier das (meth)acrylsäurehaltige Gemisch verstanden, das nach Absorption der Reaktionsgase der Propan/Propen/Acrolein- beziehungsweise Isobutan/Isobuten/Methacrolein-Oxidation in einem Absorptionsmittel und anschließender Abtrennung des Absorptionsmittels anfällt beziehungsweise das durch fraktionierende Kondensation der Reaktionsgase gewonnen wird.

Selbstverständlich kann auch Rein-(Meth)acrylsäure eingesetzt werden mit beispielsweise folgender Reinheit:

| | |
|---|---|
| (Meth)acrylsäure | 99,7 - 99,99 Gew% |
| Essigsäure | 50 - 1000 Gew.ppm |
| Propionsäure | 10 - 500 Gew.ppm |
| Diacrylsäure | 10 - 500 Gew.ppm |
| Wasser | 50 -1000 Gew.ppm |
| Aldehyde | 1 - 500 Gew.ppm |
| Inhibitoren | 1 - 300 Gew.ppm |
| Maleinsäure(-anhydrid) | 1 - 200 Gew.ppm |

Die eingesetzte Rein-(Meth)acrylsäure ist in der Regel stabilisiert mit 100 - 300 ppm Hydrochinonmonomethylether oder anderen Lagerstabilisatoren in Mengen, die eine vergleichbare Stabilisierung ermöglichen.

Unter reiner bzw. vorgereinigter (Meth)acrylsäure wird allgemein (Meth)acrylsäure verstanden, deren Reinheit mind. 99,5 Gew% beträgt und die im wesentlichen frei von den aldehydischen, anderen carbonylhaltigen und hochsiedenden Komponenten ist.

Unter O-Vinylverbindungen oder N-Vinylverbindungen wird ein heterosubstituiertes Vinylmonomeres verstanden, das als Heteroatom an der Vinyl-Gruppe Sauerstoff oder Stickstoff trägt. Als heterosubstituierte Vinylmonomere kommen beispielsweise Vinylcarbonsäureester wie Vinylacetat, Vinylpropionat oder Vinylbutyrat, Vinylether wie Methylvinylether, Ethylvinylether oder Butylvinylether, Triethylenglykoldivinylether, Hydroxyalkylvinylether wie Hydroxybutylvinylether und Cycloalkylvinylether wie Cyclohexylvinylether, ferner Vinylcarbazole, Vinylpyrrolidone, Vinylphthalimide, Vinylcaprolactame, Vinylimidazole und Vinylformamid in Betracht.

Bei einer weiteren Ausführungsform der erfindungsgemäßen Zusammensetzung fällt mindestens eine polymerisierbare Verbindung unter die allgemeine Formel (II)

H₂C=CH―X―R¹ (II),

wobei die Symbole die folgende Bedeutung haben
- X: O oder NR²,
- R¹: 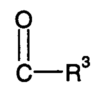 oder R³

- R²: Wasserstoff oder C₁-C₄-Alkyl oder gemeinsam mit R³ eine C₃-, C₄- oder C₅-Alkylenbrücke oder Alkenylenbrücke bildend, in welchen bis zu zwei nichtbenachbarte CH₂-Gruppen durch NH, N(C₁-C₄-Alkyl), N(C₆-C₁₀-Aryl) oder Sauerstoff und bis zu zwei nicht-benachbarte CH-Gruppen durch N ersetzt sein können und
- R³: Wasserstoff oder eine einfach oder mehrfach Hydroxy-substituierte oder Vinyloxy-substituierte oder unsubstituierte C₁-C₁₆-Alkyl-, C₆-C₁₆-Cycloalkyl- oder C₁-C₄-Alkyl-C₆-C₁₂-cycloalkylgruppe oder zusammen mit R² eine C₃-, 6₄- oder C₅-Alkylenbrücke oder Alkenylenbrücke darstellend, in welchen bis zu zwei nichtbenachbarte CH₂-Gruppen durch NH, N(C₁-C₄-Alkyl), N(C₆-C₁₀-Aryl) oder Sauerstoff und bis zu zwei nicht-benachbarte CH-Gruppen durch N ersetzt sein können.

Bei den polymerisierbaren Verbindungen der allgemeinen Formel (II), die in der erfindungsgemäßen Zusammensetzung enthalten sein können, bedeutet X bevorzugt Sauerstoff. Vinylether, in denen R¹ eine C₁-C₄-Alkylgruppe, also Methyl, Ethyl, n-Propyl, iso-Propyl, n-Butyl, iso-Butyl, sec-Butyl oder tert-Butyl ist, stellen bevorzugte polymerisierbare Verbindungen dar. Ferner sind Vinylether bevorzugt, bei denen R¹ eine C₁-C₄-Hydroxyalkylgruppe oder eine C₁-C₄-Alkylgruppe, die einfach Vinyloxysubstituiert ist, darstellt.

Weitere Ausführungsformen der erfindungsgemäßen Zusammensetzung sind solche, die als polymerisierbare Verbindung Triethylenglykoldivinylether (DVE), 4-Hydroxybutylvinylether und/oder Cyclohexylvinylether enthalten. Besitzt X die Bedeutung NR₂, so ist R¹ vorzugsweise eine CO-R³-Gruppe.

Als Reste R³ kommen neben Wasserstoff und den genannten C₁-C₄-Alkylgruppen auch solche Reste in Betracht, die zusammen mit NR2 einen gesättigten oder ungesättigten 5- bis 7- gliedrigen Ring bilden. Beispiele solcher Ringsysteme sind solche der allgemeinen Formel (III)

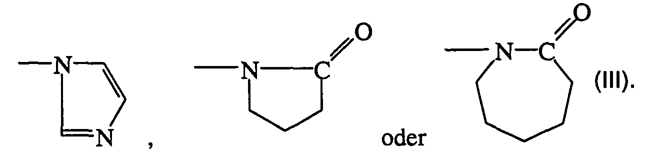

Bei einer weiteren Ausführungsform der erfindungsgemäßen Zusammensetzung stellt X-R¹ NH₂COCH₃, N-Imidazolyl, N-Pyrrolidinonyl oder N-Caprolactamyl dar.

Unter den Estern der (Meth)acrylsäure sind C₁- bis C₈-Alkylester wie der Methyl-, der Butyl- oder der 2-Ethylhexylester bevorzugt geeignet.

Die erfindungsgemäße Zusammensetzung kann in einer weiteren Ausführungsform aus TCD-Dimethylol und polymerisierbarer Verbindung bestehen, wobei für die Herstellung der polymerisierbaren Verbindung typische Verunreinigungen und Stabilisatoren im Spurenbereich wie vorstehend beispielsweise für Roh-(Meth)acrylsäure angegeben, mitumfasst sein sollen.

In einer weiteren Ausführungsform wird der erfindungsgeinäßen Zusammensetzung 1 bis 30 Gew.-%, bevorzugt 5 bis 20 Gew.-%, eines Lösungsmittels zugefügt.

Als Lösungsmittel kommen C₁- bis C₈-Alkohole, Ether, C₁- bis C₁₄-Kohlenwasserstoffe, wie beispielsweise Methanol, Ethanol, iso-Propanol, Butanol, 2-Ethylhexanol, Dioxan, Tetrahydrofuran, Ethylenglykoldimethylether, Cyclohexan und Pentan in Frage, bevorzugt sind Methanol, Butanol, 2-Ethylhexanol, Tetrahydrofuran und Ethylenglykoldimethylether.

Die Herstellung der erfindungsgemäßen Zusammensetzung erfolgt durch Mischen des TCD-Dimethylols mit polymerisierbarer Verbindung und gegebenenfalls Lösungsmittel bevorzugt unter Zuhilfenahme üblicher Rühreinrichtungen. Tricyclodecandimethylol ist als Handelsprodukt beispielsweise von der Firma Celanese Chemicals Europe GmbH, Lurgiallee 14, 60439 Frankfurt a. M. erhältlich.

Die erfindungsgemäße Zusammensetzung erspart Polymerherstellern, die Tricyclodecandimethylol verwenden wollen, beispielsweise zur Herstellung von Acrylaten oder Epoxidharzen das zeit- und kostenaufwendige Aufschmelzen der hochviskosen Substanz.

Die Erfindung wird durch die nachfolgenden Beispiele näher beschrieben.

### Beispiele

### Viskositätsmessung

Die Messung der Viskositäten erfolgte mit einem Rotationsviskosimeter nach DIN 53018. Es wurde das Rotoviso RV20 der Firma Haake, Messsystem M5, Messgeometrie MVI und MVII verwendet.

### Beispiel 1: Handelsübliches TCD-Dimethylol

Die Viskositäten von handelsüblichem TCD-Dimethylol wurden bei 50 und 75°C bestimmt. Bei 50°C wurde eine Viskosität von 16500 mPas, bei 75°C eine Viskosität von 1100 mPas gemessen

### Beispiel 2: Erfindungsgemäße Zusammensetzung

TCD-Dimethylol, Acrylsäuremethylester und gegebenenfalls Methanol werden in den in Tabelle 1 angegebenen Gewichtsverhältnissen miteinander vermischt. Die Werte für die Viskositäten bei 25°C sind ebenfalls Tabelle 1 zu entnehmen.

**Tabelle 1:**

| Beispiel | TCD-Dimethylol [g] | Acrylsäuremethylester [g] | Methanol [g] | Viskosität [mPas] |
|---|---|---|---|---|
| 1 | 90 | 10 | 0 | 8340 |
| 2 | 90 | 5 | 5 | 3930 |
| 3 | 80 | 20 | 0 | 687 |
| 4 | 80 | 10 | 10 | 298 |
| 5 | 70 | 30 | 0 | 122 |
| 6 | 70 | 15 | 15 | 51 |

## Patentansprüche

1. Zusammensetzung enthaltend Tricyclodecandimethylol und mindestens eine polymerisierbare Verbindung in einem Gewichtsverhältnis von 100:1 bis 1:1.

2. Zusammensetzung nach Anspruch 1, **dadurch gekennzeichnet, dass** die polymerisierbare Verbindung ausgewählt ist aus (Meth)acrylsäure, deren Estern, O- und N-Vinylverbindungen, Alkylenoxiden und Epichlorhydrin.

3. Zusammensetzung nach Anspruch 1 oder 2, **dadurch gekennzeichnet, dass** Tricyclodecandimethylol und die polymerisierbare Verbindung in einem Gewichtsverhältnis von 20:1 bis 3:1 vorliegen.

4. Zusammensetzung nach einem der Ansprüche 1 bis 3, **dadurch gekennzeichnet, dass** zusätzlich ein Lösungsmittel enthalten ist.

5. Zusammensetzung nach einem der Ansprüche 1 bis 4, **dadurch gekennzeichnet, dass** das Lösungsmittel ausgewählt ist aus Tetrahydrofuran, Methanol, Butanol, Ethylhexanol und Ethylenglykoldimethylether.

6. Verwendung der Zusammensetzung nach einem der Ansprüche 1 bis 5, zur Herstellung von Acrylat- und Epoxidharzen.
